(19) Europäisches Patentamt
European Patent Office
Office européen des brevets

(11) Numéro de publication: **0 177 382**
**A1**

(12)
# DEMANDE DE BREVET EUROPEEN

(21) Numéro de dépôt: 85401666.4

(22) Date de dépôt: 21.08.85

(51) Int. Cl.⁴: C 07 H 21/00
C 12 Q 1/68, C 07 H 19/16

(30) Priorité: 22.08.84 FR 8413096

(43) Date de publication de la demande:
09.04.86 Bulletin 86/15

(84) Etats contractants désignés:
AT BE CH DE GB IT LI LU NL SE

(71) Demandeur: INSTITUT PASTEUR
25-28, rue du Docteur Roux
F-75724 Paris Cedex 15(FR)

(71) Demandeur: Etablissement Public dit: CENTRE NATIONAL DE LA RECHERCHE SCIENTIFIQUE (CNRS)
15, Quai Anatole France
F-75700 Paris(FR)

(72) Inventeur: Huynh-Dinh, Tam
32 rue Paul Déroulède
F-78290 Croissy sur Seine(FR)

(72) Inventeur: Langlois d'Estaintot, Béatrice
3 rue Jacques Offenbach
F-75016 Paris(FR)

(72) Inventeur: Zakin, Mario
22 rue Victor Hugo
F-92700 Colombes(FR)

(72) Inventeur: Duchange, Nathalie
67 rue de la Croix Nivert
F-75015 Paris(FR)

(72) Inventeur: Igolen, Jean
4 rue Croix Mathurine
F-78320 Le Mesnil St Denis(FR)

(74) Mandataire: Peaucelle, Chantal et al,
S.C. Ernest Gutmann - Yves Plasseraud 67, boulevard Haussmann
F-75008 Paris(FR)

(54) **Sonde mixte et ses applications, son précurseur, leur préparation et dérivés comportant une base modifiée pour cette préparation.**

(57) L'invention a notamment pour objet un précurseur de sonde constitué d'un fragment d'oligonucléotide de structure prédéterminée par l'application envisagée, dont les fonctions réactives sont éventuellement protégées et qui est éventuellement fixé à un support, caractérisé en ce qu'une partie au moins des bases pyrimidiques y est remplacée par des groupes X et/ou une partie au moins des bases puriques y est remplacée par des groupes Y, les groupes X et Y répondant respectivement aux formules :

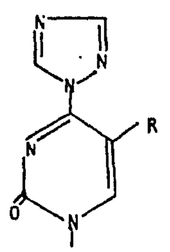

X

et

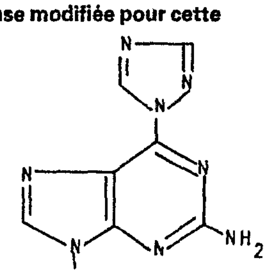

Y

R représentant un atome d'hydrogène ou un groupe méthyle, et la sonde mixte obtenue par dismutation, dans ce précurseur de X en U+C ou T+${}^{Me}C$ et de Y en G+${}^{NH_2}A$.

Application aux analyses biologiques et à l'extraction de fragments d'ARN ou d'ADN.

EP 0 177 382 A1

Sonde mixte et ses applications, son précurseur, leur préparation et dérivés comportant une base modifiée pour cette préparation.

L'invention concerne des séquences d'oligonucléotides pouvant remplacer les sondes mixtes pour la détection et l'isolement de séquences de nucléotides, notamment d'ARN messagers ou d'ADN complémentaires, leurs précurseurs et leur préparation, ainsi que leurs utilisations.

Plus précisément, l'invention a pour objet des séquences d'oligonucléotides du type susindiqué comprenant des bases modifiées, leur préparation à partir de précurseurs comprenant des bases qui, par dismutation, conduisent aux bases modifiées en question, la préparation de ces précurseurs, ainsi que l'utilisation des sondes obtenues.

Il est maintenant bien connu des spécialistes d'avoir recours pour certaines analyses, notamment pour la détection de séquences nucléotidiques particulières, telles que des séquences d'ADN ou d'ARN et l'isolement de telles séquences, à des techniques basées sur l'hybridation entre une séquence déterminée de nucléotides utilisée en tant que sonde et une séquence de nucléotides complémentaires de ceux de la sonde, éventuellement contenue dans une composition renfermant des acides nucléiques. La mise en oeuvre de ces techniques, en particulier l'obtention de sondes sensibles et s'hybridant de façon stable avec les séquences complémentaires, pose encore des problèmes, notamment au niveau de leur synthèse.

Ainsi, l'un des problèmes les plus fréquents posés en génie génétique est celui de la "pêche" d'ARN messagers par des oligodésoxynucléotides synthétiques, c'est-à-dire la détection, l'isolement et l'analyse de séquences d'ARN messagers par hybridation avec des fragments d'ADN complémentaires (cADN). Cette technique est basée sur l'appariement des paires A-T (2 liaisons hydrogène) et G-C (3 liaisons hydrogène).

La conception et la synthèse de ces sondes sont rendues compliquées par la dégénérescence du code génétique : 
32 à part la méthionine (AUG) et le tryptophane (UGG)

qui nécessitent un seul codon. tous les autres acides aminés sont traduits à partir de 2 à 6 codons.

Jusqu'à maintenant. à partir d'une séquence protéique déterminée. pour obtenir des sondes. on a utilisé plusieurs stratégies :

a) choisir dans une séquence G de préférence à A.et T de préférence à C. ce qui donne à l'hybridation soit une paire stable, soit un mauvais appariement G-T appelé de façon générale et dans ce qui suit "mismatch" G-T. Cette méthode a été appliquée avec succès pour la synthèse de cADN codant la relaxine de rat [P.Hudson et coll.. Nature. (1981). 291.127] mais est difficilement généralisable en raison de son manque de sensibilité notamment.

b) préparer toutes les séquences possibles avec les ambiguités correspondantes [B.E. Noyes et coll.. Proc. Natl. Acad. Sci.. USA. (1979). 76. 1770 et M. Mevarech et coll.. J. Biol. Chem.. (1979).254. 7472]. ce qui peut nécessiter un énorme travail de synthèse.

c) synthétiser des "sondes mixtes" ("mixed probes") avec toutes les ambiguités sur la même séquence. Cette technique a donné de bons résultats pour la β-globine[R.B. Wallace et coll.. Nucleic Acids Research. (1981). 9.879 ]. par exemple. mais représente pour le chimiste organicien un côté "frustrant" vu la complexité du mélange obtenu et le nombre de trimères mis en jeu.et au surplus ne donne pas toujours des résultats satisfaisants en raison notamment des réactivités différentes des différents trimères mis en jeu au niveau de chaque ambiguité. ce qui a pour conséquence pratique que l'on n'a pas de certitude quant à la composition exacte du mélange obtenu.

L'invention remédie. au moins en partie. aux inconvénients des techniques antérieures en fournissant une sonde qui :

- tout en ayant les caractères d'une sonde mixte, constituée d'un mélange complexe, est synthétisée comme une sonde unique, ce qui correspond à un important gain de temps et d'argent ;

- présente, par rapport notamment aux sondes définies sous a) ci-dessus, une meilleure sensibilité dans la détection après hybridation avec des séquences complémentaires et une meilleure stabilité de l'hybride ainsi formé (température de fusion plus élevée) ;

et au surplus, comme il sera vu plus loin,

- offre la possibilité d'être utilisée avec des méthodes d'identification faisant intervenir des réactions immunologiques voire immuno-enzymatiques à la place des détections radioactives relativement peu sensibles et présentant les inconvénients bien connus, utilisées jusqu'à présent.

La présente invention repose sur la découverte selon laquelle il est possible d'obtenir, par dismutation d'une séquence unique d'oligonucléotide, dans des conditions bien particulières, une séquence mixte comportant, à chaque fois que se pose une ambiguité au niveau d'une pyrimidine, une quantité équimoléculaire de méthyl-5 cytosine ($^{Me}C$) et de thymine (T) ou une quantité équimoléculaire d'uracile (U) et de cytosine (C), et à chaque fois que se pose une ambiguité au niveau d'une purine, une quantité équimoléculaire de guanine (G) et d'amino-2 adénine ($^{NH_2}A$).

La séquence mixte ainsi obtenue qui contient certaines bases modifiées $^{Me}C$ et/ou $^{NH_2}A$ présente toutes les caractéristiques des séquences mixtes préparées par les moyens conventionnels exposés plus haut.

On avait déjà proposé [W.L. Sung, Nucleic Acids Research, (1981), 9,6139] d'introduire un groupe méthyl-5 cytosine dans une séquence d'ADN en traitant par l'ammoniaque une séquence d'ADN contenant le groupe triazolo-4 thymine, selon la réaction :

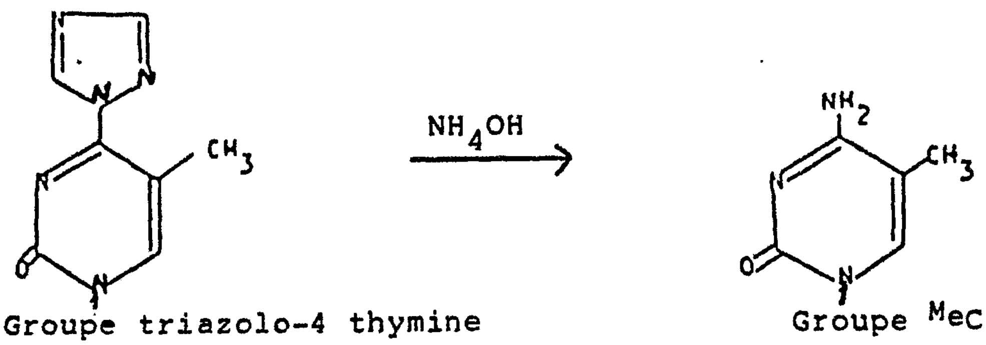


Groupe triazolo-4 thymine Groupe $^{Me}C$

Cette réaction fournit, de manière quasi-quantitative, une séquence contenant uniquement des groupes méthyl-5 cytosine à la place des groupes triazolo-4 thymine.

On a maintenant établi que l'on pouvait, en travaillant dans des conditions bien déterminées qui seront explicitées plus loin, dismuter, en quantités équimoléculaires, dans une séquence d'oligonucléotide, un groupe triazolo-4 uracile en groupes uracile et cytosine, un groupe triazolo-4 thymine en groupes thymine et méthyl-5 cytosine et un groupe triazolo-4 guanine en groupes guanine et amino-2 adénine, selon les schémas respectifs 1 et 2 suivants :

1.

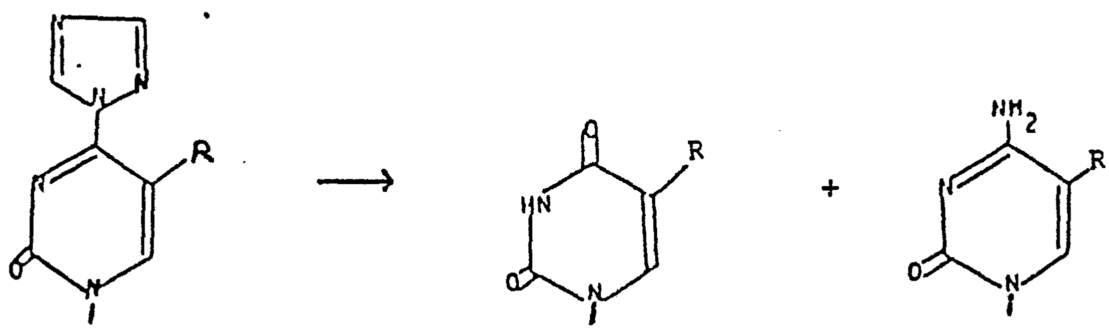

R= H U + C

R= $CH_3$. T + $^{Me}C$

2.

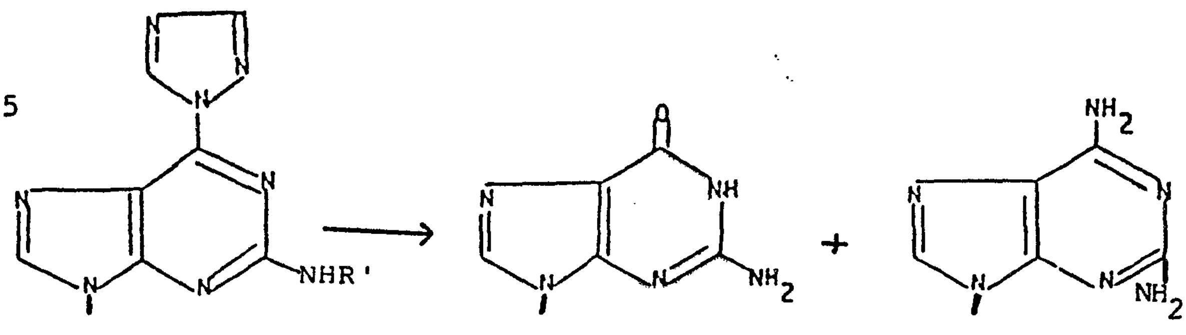

G $NH_{2A}$

R' étant un groupe protecteur du groupe amino.

Selon un premier aspect, l'invention a pour objet un précurseur de sonde constitué d'un fragment d'oligonucléotide de structure prédéterminée par l'application envisagée, dont les fonctions réactives sont éventuellement protégées et qui est éventuellement fixé à un support, caractérisé en ce qu'une partie au moins des bases pyrimidiques y est remplacée par des groupes X et/ou une partie au moins des bases puriques y est remplacée par des groupes Y, les groupes X et Y répondant respectivement aux formules

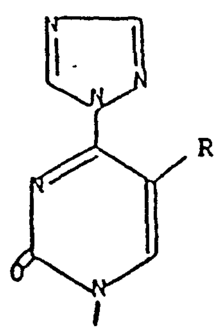

X

et

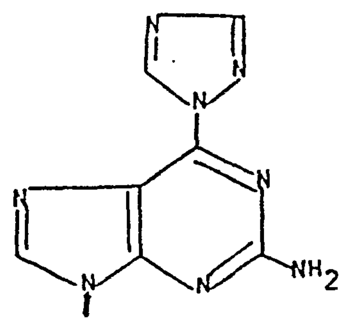

Y

R représentant un atome d'hydrogène ou un groupe méthyle.

Après dismutation selon l'invention, séparation éventuelle du support et élimination des groupes protecteurs, ce précurseur conduit à une sonde comportant, à la place des groupes X, en parties égales, soit des groupes U et C (R=H), soit des groupes T et $^{Me}C$ ($R=CH_3$) et à la place des groupes Y, en parties égales, des groupes G et $^{NH_2}A$.

Il est particulièrement intéressant d'utiliser un précurseur de ce type pour préparer des sondes mixtes contenant des ambiguités au niveau d'une base pyrimidique et/ou au niveau d'une base purique, c'est-à-dire des sondes mixtes contenant les ambiguités U/C ou T/C et/ou G/A.

Selon un mode préféré de réalisation, l'invention a pour objet un précurseur de sonde constitué d'un fragment d'oligonucléotide de structure prédéterminée par l'application envisagée, dont les fonctions réactives sont éventuellement protégées et qui est éventuellement fixé à un support, caractérisé en ce que chaque ambiguité au niveau d'une base pyrimidique y est remplacée par un groupe X et chaque ambiguité au niveau d'une base purique y est remplacée par un groupe Y, les groupes X et Y étant tels que définis plus haut.

Le précurseur selon l'invention peut être préparé selon tout procédé classique de préparation de fragments d'oligonucléotides en remplaçant une partie au moins des bases pyrimidiques par des groupes X et/ou une partie au moins des bases puriques par des groupes Y.

Avantageusement, la préparation est effectuée en phase solide, essentiellement par fixation d'un monomère, dimère ou trimère par son extrémité 3' sur l'extrémité 5' du nucléotide terminal 3' de la séquence désirée, fixé sur un support solide, puis fixations successives de monomères,

dimères et/ou trimères, à chaque fois sur le nucléotide terminal 5'de la chaîne obtenue, jusqu'à obtention de la fraction d'oligonucléotide désirée.

Une telle préparation peut être effectuée selon les indications données par B.L. Gaffney èt coll. dans Nucleic Acids Research, (1982), 10, 435 et Tetrahedron, (1984), 40, 3.

On entend ici par "monomère", "dimère", ou "trimère" des groupes constitués respectivement de 1,2 ou 3 acides nucléiques, identiques ou différents, devant entrer dans la constitution de la sonde.

Il va de soi qu'au moment de la synthèse, les fonctions réactives de ces groupes dont la participation à la réaction envisagée n'est pas souhaitée, doivent être protégées au moyen de groupes protecteurs pouvant être ensuite éliminés dans des conditions non dégradantes pour la sonde que l'on souhaite préparer.

A titre d'exemples non limitatifs, la préparation de monomères, dimères et trimères portant des groupes protecteurs de ce type peut être effectuée selon les indications de K. Itakura et coll. dans Nucleic Acids Research, (1980), 8, 5507.

Les groupes acide nucléique modifié contenant, en tant que base, la base X ou la base Y, utilisables en tant que monomères dans la synthèse du précurseur de sonde selon l'invention ou entrant dans la préparation des dimères ou trimères utilisables dans cette synthèse peuvent être préparés selon les méthodes connues de préparation de monomères habituellement utilisées dans de telles synthèses, en remplaçant les bases usuelles pour un groupe X ou Y selon le cas.

A titre d'exemples non limitatifs, il est indiqué que pour la préparation d'un fragment d'ADN :

- le monomère contenant la base modifiée X (avec $R = CH_3$) peut être la O-diméthoxytrityl-5' O-(chloro-2 phényl cyanoéthyl) phosphate-3' triazolo-4 thymidine, de formule

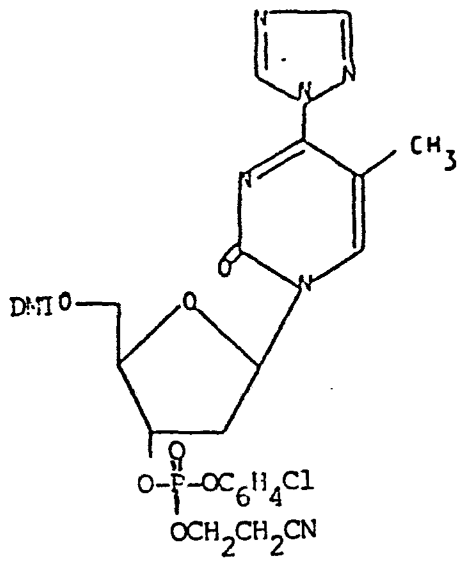

DMT=diméthoxytrityle

qui peut être préparée, comme décrit plus en détail dans l'exemple préliminaire 1 qui suit, par réaction de la diméthoxytrityl-5' thymidine (qui est un produit commercial) avec le triazole puis le 2-chlorophényl phosphorodichloridate et enfin le cyanoéthanol et

- le monomère contenant la base modifiée Y peut être la N-isobutyryl-2 O-diméthoxytrityl-5' O-(chloro-2 phényl cyanoéthyl) phosphate-3' triazolo-4 désoxy-2' guanosine, de formule

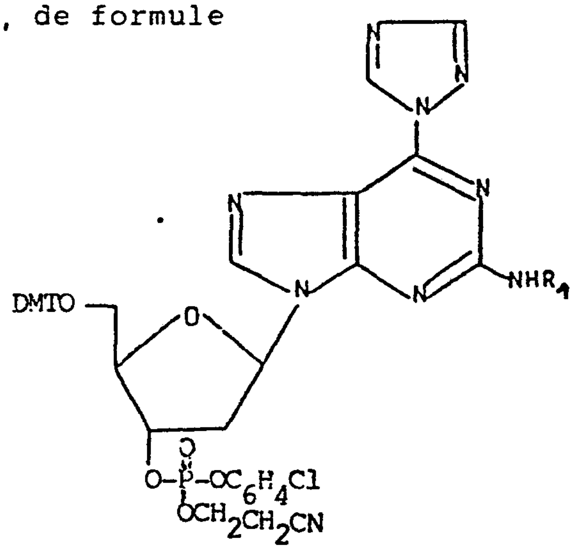

$R_1$=isobutyryle

qui peut être préparée, comme décrit plus en détail dans les exemples préliminaires 2a et 2b qui suivent, par réaction de la N-isobutyryl-2 O-diméthoxy-trityl-5' désoxy-2' guanosine ou du phosphate correspondant avec le triazole, puis le 2-chlorophényl phosphorodichloridate et enfin le cyanoéthanol.

Les monomères contenant la base X ou la base Y et destinés à la préparation de fragments d'ARN peuvent être préparés en analogie à ce qui est décrit ci-dessus.

Selon un second aspect, l'invention vise les sondes mixtes telles qu'obtenues par déblocage des précurseurs décrits ci-dessus, avec dismutation, de préférence en proportions égales, des nucléotides contenant des groupes X et/ou Y tels que définis plus haut qu'ils contiennent.

Selon l'invention, on a entre autres établi, et cela est surprenant, qu'en débloquant les groupes protecteurs dans des conditions bien déterminées, non seulement on pouvait obtenir, en proportions égales, d'une part les produits de dismutation du groupe X et d'autre part les produits de dismutation du groupe Y, mais encore que ces conditions étaient les mêmes dans les deux cas.

Ces conditions consistent essentiellement à, successivement :

a) traiter le précurseur selon l'invention par un mélange molaire de pyridine aldoxime (PAO) dans la tétraméthyl guanidine (TMG), additionné d'ammoniaque concentrée (à 20 % au moins) dans la proportion de 1 volume de mélange PAO-TMG pour 10 volumes d'ammoniaque, à la température ambiante, pendant une nuit;

b) traiter le mélange obtenu sous a), évaporé à sec, par l'ammoniaque concentrée, pendant environ 5 heures à environ 50°C ; et

c) traiter le mélange obtenu sous b), évaporé à sec, par l'éthylène diamine, pendant environ 72 heures à la température ambiante.

Il est intéressant de noter que ce traitement "débloque" également le groupe $NH_2$ de l'adénine.

Cela ne pouvait être déduit des essais de "déblocage" effectués d'une part avec 1 mg de nucléotide renfermant la base X et d'autre part avec 1 mg de nucléotide renfermant la base Y et qui sont résumés dans le tableau suivant dans lequel les abréviations sont les mêmes que précédemment, $NH_4OH$ représentant l'ammoniaque concentrée (c'est-à-dire à 20 % au moins) et t.a. la température ambiante.

<u>X</u>

- 1. 100 µl TMG-PAO 0,3 M, 15h, t.a.; 2. 500 µl $NH_4OH$ 5 h, 50°C → T (99 %).
- 1. 1 ml dioxanne-$NH_4OH$ (3-1), 3h, t.a.; 2. 500 µl $NH_4OH$ 5 h, 50°C → $^{Me}C$ (99 %).
- 1. 300 µl TMG-PAO 0,3 M + 3 ml $NH_4OH$, 24 h, 40°C → 35 % T + 65 % $^{Me}C$
- 1. 300 µl TMG-PAO 0,3 M, 4 h, t.a.; 2. 3 ml $NH_4OH$ 15 h, 50°C → 93 % T + 6 % $^{Me}C$.

<u>Y</u>

- 1. 100 µl TMG-PAO 0,3 M, 15 h, t.a.; 2. 500 µl $NH_4OH$ 5 h, 50°C → 100 % G
- 1. 400 µl dioxanne-$NH_4OH$(3-1) 3 h, t.a.; 2. 500 µl $NH_4OH$ 48 h, 50°C → 100 % $A^{NHiBu}$
- 1. 400 µl dioxanne-$NH_4OH$(3-1) 3 h, t.a.; 2. 500 µl $NH_4OH$ 48 h, 50°C;
  3. 1 ml éthylène diamine, 72 h, t.a. → 100 % $A^{NH_2}$
- 1. 100 µl TMG-PAO 0,3 M + 1 ml $NH_4OH$ 16 h, t.a.;

2. 500 µl $NH_4OH$ 98 h, 65°C → 55 % G + 31 % $A^{NHiBu}$ + 13 % $A^{NH_2}$

Pour la plupart des applications, la sonde selon l'invention comporte moins de 20 nucléotides par fragment. Toutefois, pour certaines applications particulières, il peut être souhaitable de disposer de fragments plus "longs" d'oligonucléotides. Compte tenu de l'absence d'ambiguité au moment de la préparation du précurseur, il n'exite en principe aucune limitation "technique" à la préparation de fragments plus longs. Ainsi, on a montré qu'il était possible d'obtenir des séquences comportant plus de 40 nucléotides,ce qui permet d'obtenir une meilleure sensibilité.

Bien qu'il soit préférable, comme indiqué plus haut, que toutes les ambiguités sur des bases pyrimidiques soient remplacées par les produits de dismutation de nucléotides contenant la base X et que toutes les ambiguités sur des bases puriques soient remplacées par les produits de dismutation de nucléotides contenant la base Y, l'invention comprend également les sondes dans lesquelles une partie des ambiguités ont été remplacées par l'un des nucléotides de l'ambiguité, notamment par un nucléotide contenant G dans le cas d'une ambiguité G/A et par un nucléotide contenant T pour une ambiguité T/C. Il faut rappeler toutefois que ce choix est susceptible d'introduire des "mismatchs".

L'invention comprend également les précurseurs et les sondes mixtes comprenant, outre les groupes amino-2 adénine éventuellement introduits par dismutation du groupe Y, des groupes adénine modifiés de telle sorte qu'ils soient capables de donner trois liaisons hydrogène avec la thymine ou l'uracile,notamment des groupes amino-2 adénine remplaçant soit un groupe adénine sur lequel il n'y a pas d'ambiguité, soit une ambiguité G/A, comme cela est expliqué plus en détail dans la demande de brevet

français déposée le même jour, par les mêmes déposants, sous le titre "sondes comportant des groupements adénine modifiée, leur préparation et leurs utilisations.

Quel que soit leur mode d'introduction dans la fraction nucléotidique, les groupes adénine modifiée confèrent à l'hybride formé par la sonde ainsi obtenue une meilleure stabilité en raison de l'augmentation de son point de fusion. On sait en effet que lorsqu'on remplace dans une séquence nucléotidique une désoxy-2' adénosine (A) par une amino-2 désoxy-2' adénosine (A*), cela conduit, dans l'hybride formé par cette séquence, à la transformation d'une paire AT présentant deux liaisons hydrogène, en une paire A*T présentant trois liaisons hydrogènes et qui a une stabilité équivalente à celle d'une paire CG [R.C. Comtor et P.R. Schimmel, Biophysical Chemistry, W.H. Freeman, San Francisco, (1980)].

Par ailleurs, et c'est là un des avantages particuliers des sondes selon l'invention qui contiennent des groupes adénine modifiée, les demandeurs ont établi que la présence de tels groupes modifiés , notamment par des groupes $-NH_2$ sur le sommet C-2 du cycle pyrimidine favorise la configuration gauche, appelée configuration Z, des hybrides formés à partir des séquences de ce type, par rapport à la configuration usuelle B et que dans la configuration Z, les groupes adénine modifiée sont plus accessibles à des réactifs extérieurs.

Ceci permet leur repérage, notamment par des anticorps spécifiques de ces bases, de préférence monoclonaux, alors que la configuration B ne permet que difficilement, voire même empêche, l'accession des réactifs aux bases.

La sonde selon l'invention, lorsqu'elle comporte des groupes adénine modifiée, notamment amino-2 adénine, introduits par dismutaiton des groupes Y de son précurseur et/ou en remplacement d'une partie au moins des groupes adénine peut donc être avantageusement utilisée dans des techniques d'analyse ou d'extraction, notamment d'ARN messagers ou d'ADN complémentaires, faisant appel à des mises en évidence comportant la formation de complexes immuns et mettant éventuellement en jeu des réactions immunoenzymatiques.

Pour son utilisation, la sonde obtenue selon l'invention est avantageusement marquée, en vue de son repérage après hybridation, selon un technique usuelle dans le domaine des analyses biologiques.

Ainsi, elle peut être marquée radioactivement notamment au $^{32}P$ au moyen de [$\gamma$- $^{32}P$]ATP en présence de polynucléotide kinase, comme décrit dans les exemples qui suivent.

L'invention vise également une sonde telle que définie plus haut, marquée radioactivement, notamment par $^{32}P$.

L'invention vise enfin l'utilisation de la sonde définie précédemment sous ses différents aspects dans le domaine des analyses et extractions biologiques, notamment de détection et d'extraction d'ARN messagers et d'ADN complémentaires, au moyen d'une hybridation.

Il sera décrit ci-après, à titre d'exemple, l'application de l'invention à la résolution du problème posé par l'isolement de l'ADN complémentaire (cADN) de l'antithrombine III.

La séquence d'aminoacides prise en considération est:

$$\overset{251}{\text{Met}} - \text{Met} - \text{Tyr} - \text{Gln} - \text{Glu} - \overset{256}{\text{Gly}}\ ,$$

ce qui correspond à la séquence de mRNA :

$$5'^{OH}\ \text{AUG}\quad \text{AUG}\quad \text{UA}_{C}^{U}\quad \text{CA}_{G}^{A}\quad \text{GA}_{G}^{A}\quad \text{GG}\begin{smallmatrix}A\\C\\G\\U\end{smallmatrix}\quad 3'_{OH}$$

La sonde mixte correspondante s'écrit :

$$3'_{OH}\text{TAC}\quad \text{TAC}\quad \text{AT}_{G}^{A}\quad \text{GT}_{C}^{T}\quad \text{CT}_{C}^{T}\quad \text{C}\ 5'\ ^{OH}$$

La séquence de cADN de l'antithrombine III déterminée après séquençage s'est révélée être :

$$5'^{OH}\text{ATG}\quad \text{ATG}\quad \text{TAC}\quad \text{CAG}\quad \text{GAA}\quad \text{G}\ 3'_{OH}\ \ldots$$

On a préparé deux sondes destinées à s'hybrider avec le cADN de l'antithrombine III, à savoir :

1° la sonde mixte comportant les trois ambiguités (mélange de 8 hexadécamères) a été construite à partir de triplets en phase solide, selon la technique de R.B. Wallace et coll. dans Nucleic Acids Research, (1981), 9, 879, suivant le schéma :

| CTT / CCT | CTT / CCT | GAT / GGT | ACA | TCA | T~ | ← Support solide |
|---|---|---|---|---|---|---|

ce qui a nécessité l'emploi de 8 trimères ;

2° la sonde modifiée selon l'invention qui a été obtenue à partir du précurseur selon l'invention, lui-même synthétisé, selon la méthode de H. Ito et coll., Nucleic Acids Research (1982), 10, 1755. à l'aide de monomères et de dimères, suivant le schéma:

C|X|TC|X|TG|Y|TA|CA|TC|A|T~

Au déblocage, dans des conditions bien définies, ce précurseur a donné une sonde composée des huit séquences :

```
          T     T      G
       C   TC    TG    TACATCAT
        MeC   MeC   NH2A
```

Ces deux sondes on été marquées au $^{32}P$ et ont servi à analyser une "banque" contenant du cADN de l'antithrombine III. L'une et l'autre ont permis d'isoler le cADN dont la structure a été prouvée par séquençage.

PARTIE EXPERIMENTALE

Exemples préliminaires : préparation des nucléotides contenant les bases X et Y

1. Synthèse de la O-diméthoxyltrityl-5' O-(chloro-2 phényl cyanoéthyl)phosphate-3' triazolo-4 thymidine DMT ${}^{t}T_{p}$ (nucléotide contenant la base X)

5 g, (9,2 mmoles) de diméthoxytrityl-5' thymidine sont coévaporés 2 fois avec de la pyridine. On ajoute 43 ml de pyridine puis 3,872 g (56 mmoles) de triazole. Le mélange est refroidi à 0°C et on ajoute 6,72 g (27,4 mmoles) de 2-chlorophényl phosphorodichloridate. Un léger précipité se forme. On laisse sous agitation à température ambiante pendant 40 heures. Ensuite 15,08 g (212,4 mmoles) de cyanoéthanol sont ajoutés. Après une heure trente, on ajoute 150 ml d'eau distillée et on extrait deux fois avec du dichlorométhane. La phase organique est lavée une fois au bicarbonate de sodium à 0,2 % puis à l'eau jusqu'à ce que le pH de la phase aqueuse soit neutre. La phase organique est séchée sur sulfate de sodium, évaporée à sec puis précipitée à l'éther de pétrole. Le produit obtenu est purifié par chromatographie sur colonne de silice éluée au dichlorométhane enrichi progressivement en méthanol. On obtient 1,54 g de DMT ${}^{t}T_{p}$ (rendement 20 %).

CCM : Système (dichlorométhane/méthanol : 90/10)
Rf : 0,54. Le produit est fluorescent à 310 nm

Spectre UV : (ETOH 96°)

λ max 328 nm (ε = 6 600)

λ min 292 nm (ε = 2 800)

λ 234 nm (ε = 26 400)

Spectre de masse : (spectre réalisé avec un appareil commercialisé sous la dénomination VG Micromass 305, en DCI $NH_3$ à 200°C)

303 (72,0) DMT· ; 245 (1,0) phosphate protégé ; 178 (2,5) thymine triazolée.

2. Synthèse de la N-isobutyryl-2 O-diméthoxytrityl-5' O-(chloro-2 phényl cyanoéthyl)phosphate-3' triazolo-4 désoxy-2' guanosine ou DMT $^{t}G_{p}^{Ib}$ (nucléotide contenant la base Y)

a) A partir de N-isobutyryl-2 O-diméthoxytrityl-5' désoxy-2' guanosine (DMT $G^{Ib}$)

2 g (3,13 mmoles) de N-isobutyryl-2 O-diméthoxytrityl-5' désoxy-2'guanosine sont traités de la même façon que la diméthoxytrityl-5' thymidine, avec 1,386 g (20 mmoles) de triazole, 2,464 g (10 mmoles) de 2-chlorophényl phosphorodichloridate dans 16 ml de pyridine. La réaction est totale au bout de 17 heures. On ajoute 5,4 g (76 mmoles) de cyanoéthanol. Deux heures après, le mélange réactionnel est dilué dans du dichlorométhane, lavé deux foix au bicarbonate de sodium à 5 % et une fois à l'eau distillée. La phase organique est séchée sur sulfate de sodium et évaporée à sec. Le produit obtenu est purifié par chromatographie sur colonne de silice. On obtient 1,11 g de DMT $^{t}G_{p}^{Ib}$ (rendement 38 %).

b) A partir de N-isobutyryl-2 O-diméthoxytrityl-5' phosphate-3' -désoxy-2' guanosine (DMT $^{t}G_{p}^{Ib}$)

0,5 g (0,566 mmole ) de DMT $G_{p}^{Ib}$ est traité par 251 mg (3,635 mmoles) de triazole et 0,44 g (1,79 mmoles) de 2-chlorophényl phosphorodichloridate dans 5 ml de pyridine.

Au bout de 5 heures, on traite de la même façon que précédemment et on obtient 360 mg de DMT· $^{t}G_{p}^{Ib}$ (rendement 68,2 %).

CCM :(système dichlorométhane/méthanol : 80/20)
Rf : 0,575. Le produit est fluorescent à 310nm.

Spectre UV : (EtOH 96 %)

$\lambda$ max 338 nm ($\varepsilon$ = 4 200)
$\lambda$ min 300 nm ($\varepsilon$ = 3 100)
$\lambda$ 252 nm ($\varepsilon$ = 16 800)
$\lambda$ 231 nm ($\varepsilon$ = 32 000)

Spectre de masse : spectromètre de type VG Micromass 305, EI 70 eV
303 (100) DMT ; 273 (32,5) guanine triazolée et isobutyrylée ; 200 (1,6) guanine triazolée ; 132 (1,4) guanine ;

Exemple 1 :

Synthèse de la sonde mixte n° 1 :

La sonde mixte a été "montée" à partir de 19 mg de résine T [résine portant le nucléotide T et préparée selon la technique de K. Itakura et coll. décrite dans Nucleic Acids Research (1980), 8, 5473] avec les trimères suivants :

CTT CCT GAT
\+ \+ \+ ACA TCA T 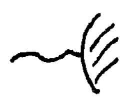
CCT CCT GGT

Rendements: 86% 83% 79% 50%

Après le dernier couplage, la résine est traitée par 500 µl d'un mélange molaire de pyridine aldoxime(PAO) dans la tétraméthyl guanidine (TMG) additionné, de 500 µl de TMG pendant une nuit. Après évaporation à sec et chauffage avec de l'ammoniaque concentrée à 50°C pendant 3 heures, le milieu réactionnel est purifié par chromatographie sur une colonne de Sephadex G-10 (dénomination commerciale de la société Pharmacia pour une colonne de polysaccharide réticulé), suivie d'une chromatographie liquide haute performance (HPLC) sur colonne en phase inverse. Le mélange d'oligonucléotides est ensuite détritylé avec l'acide acétique à 80 % pendant 5 mn, évaporé à sec (9 D.O. ; 1 D.O. = une unité de densité optique, correspondant à 20-40 µg de séquence)
et purifié par électrophorèse préparative sur gel d'acrylamide.

Exemple 2 :

Synthèse de la sonde modifiée n° 2 :

Le précurseur de la séquence n° 2 est synthétisé à partir de 40 mg de résine T, selon le schéma suivant :

C X TC X TG Y TAC AT CA T

Rendements: 86% ≤99% 92% 64% ≤99% 56% 95% 83%

Après couplage du dernier monomère, 20 mg de résine sont traités par 375 µl de mélange molaire de pyridine aldoxime (PAO) dans la tétraméthyl guanidine (TMG), additionné de 3,75 ml d'ammoniaque concentrée à la température ambiante pendant une nuit. On évapore à sec, ajoute 5 ml d'ammoniaque concentrée et chauffe 5 heures à 50°C. Après évaporation à sec, on laisse 72 heures à la température ambiante dans 2,5 ml d'éthylène diamine. Le filtrat est chromatographié sur une colonne de Sephadex G-10, purifié par HPLC sur colonne de phase inverse, et détritylé avec l'acide acétique à 80% pendant 20 mn. Le mélange brut est purifié par électrophorèse préparative.

Exemple 3 :

Marquage des sondes au $^{32}P$

On ajoute 50 µCi de [$\gamma$-$^{32}P$]ATP sur 100 pmoles de la sonde dans un volume de 4 µl + 0,5 µl de mix et 0,5 µl de polynucléotide kinase [activité 5-20 $10^3$ unités/ml Boehringer Mannheim]. On laisse une demi-heure à 37°C [T.Maniatis et coll. Molecular Cloning, Cold Spring Harbor Lab. (1982)].

La réaction est stoppée par addition de 1,3 µl de tampon au bleu de bromophénol 100 mM EDTA, 50% glycérol. Le produit marqué est purifié sur gel (0,4 x 30 X 40 cm) contenant 19 g de bisacrylamide, 19 g d'acrylamide et 10 ml de tris borate 1 M pour un volume total de 100 ml. L'électrophorèse sous programmation : 2 000 V, 43 W, 40 mA, est arrêtée quand le bleu de bromophénol atteint le tiers de la longueur de la plaque. La bande radioactive est découpée et extraite pendant une nuit dans 2 ml d'eau.

Exemple 4 : Hybridation

3 colonies contenant un cADN de l'$AT_{III}$ et 3 colonies contenant un cADN non relaté à l'$AT_{III}$ ont été repiquées en 2 exemplaires sur boîtes de milieu complet avec 50 µg/ml d'ampicilline. Les colonies ont été transférées sur 2 filtres de type Whatman 541, amplifiées 20 heures sur boîtes contenant 250 µg/ml de chloramphénicol et préparées pour l'hybridation, selon la technique décrite par J.O. Gergen et coll. dans Nucleic Acids Research,(1979),7,2115. Les filtres ont été préhybridés 2 heures à 42°C en 6 NET (1 NET = 0,15 M NaCl, 0,015 M tris HCl, pH = 7,5, 0,001 M EDTA), 0,5 % Monidet P40 (dénomination commerciale pour un détergent), 100 µg/ml de tRNA de levure et 100 µg/ml d'ADN soniqué de sperme de saumon [technique de T. Maniatris et coll. dans l'article cité plus haut]. L'hybridation a été effectuée à 42°C pendant 20 heures dans la même solution en présence de $10^6$ cpm d'oligonucléotide marqué en 5' au [$\gamma$-$^{32}$P]ATP, chaque filtre ayant été hybridé avec une des deux sondes. Les filtres ont été lavés 4 fois 15 minutes à 40°C en 6 SSC (1 SSC = 0,15 M NaCl, 0,015 M citrate de sodium, pH - 7,2), 0,1 % de dodécylsulfate de sodium (SDS). Les filtres ont été soumis à une autoradiographie.
L'hybridation est visible sur la sonde mixte 1 (1 séquence sur 8 est complémentaire du cADN) et sur la sonde modifiée 2.

Il est rappelé que le "mix" est un mélange de :

- tris(hydroxyméthyl) aminométhane (500 mM, pH 8) ;
- $MgCl_2$ (100 mM) ; et
- dithiotréitol (50 mM).

o

o o

Les nucléosides et nucléotides comportant la base Y, utilisables dans la synthèse des précurseurs selon l'invention, sont nouveaux.

Ces composés répondent à la formule générale :

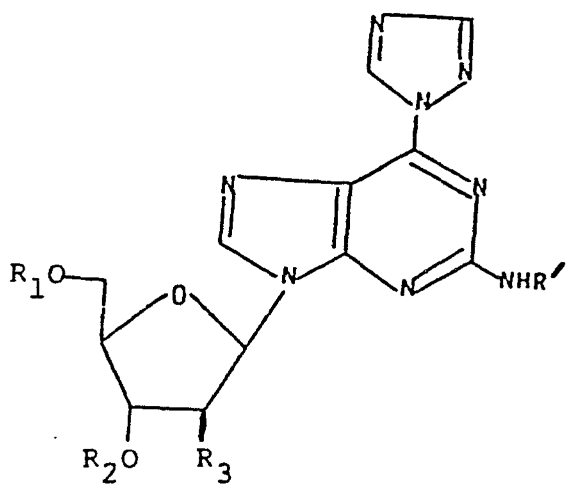

dans laquelle :

R' représente un atome d'hydrogène ou un groupe protecteur du groupe amino, notamment le groupe isobutyryle,

$R_1$ représente un atome d'hydrogène ou un groupe protecteur du groupe hydroxyle, notamment le groupe diméthoxytrityle,

$R_2$ représente un atome d'hydrogène, un groupe protecteur du groupe hydroxyle ou un groupe phosphoryle éventuellement modifié, notamment le groupe O-(chloro-2 phényl cyanoéthyl) phosphate ; et

$R_3$ représente un atome d'hydrogène, un groupe OH ou un groupe OH protégé, notamment le groupe benzoyloxy.

La préparation de ces composés peut être effectuée comme indiqué plus haut, ou par analogie avec des procédés connus de préparation de composés de structure analogue.

REVENDICATIONS

1. Précurseur de sonde constitué d'un fragment d'oligonucléotide de structure prédéterminée par l'application envisagée, dont les fonctions réactives sont éventuellement protégées et qui est éventuellement fixé à un support, caractérisé en ce qu'une partie au moins des bases pyrimidiques y est remplacée par des groupes X et/ou une partie au moins des bases puriques y est remplacée par des groupes Y, les groupes X et Y répondant respectivement aux formules :

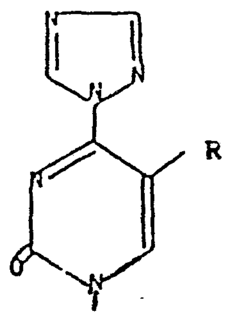

et

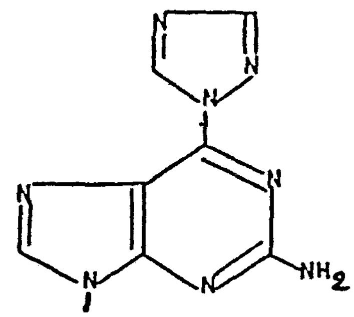

X Y

R représentant un atome d'hydrogène ou un groupe méthyle.

2. Précurseur de sonde constitué d'un fragment d'oligonucléotide de structure prédéterminée par l'application envisagée, dont les fonctions réactives sont éventuellement protégées et qui est éventuellement fixé à un support, caractérisé en ce que chaque ambiguité au niveau d'une base pyrimidique y est remplacée par un groupe X et chaque ambiguité au niveau d'une base purique y est remplacée par un groupe Y, les groupes X et Y étant tels que définis à la revendication 1.

3. Précurseur de sonde selon la revendication 1 ou 2, caractérisé en ce qu'il est constitué d'un fragment de désoxyoligonucléotide et en ce que, dans le groupe X, R représente le groupe méthyle.

4. Sonde mixte telle qu'obtenue par déblocage du précurseur selon l'une des revendications 1 à 3 avec dismutation, de préférence en proportions égales, des nucléotides contenant des groupes X et/ou Y tels que définis à la revendication 1.

5. Sonde mixte selon la revendication 4, caractérisée en ce qu'elle est en outre marquée radioactivement, notamment par $^{32}P$

6. Procédé pour la préparation du précurseur selon l'une des revendications 1 à 3, caractérisé en ce qu'il est effectué en phase solide et consiste essentiellement à fixer un monomère, dimère ou trimère par son extrémité 3' sur l'extrémité 5' du nucléotide terminal 3' de la séquence désirée, fixé sur un support solide, puis à fixer successivement les monomères, dimères et/ou trimères suivants, à chaque fois sur le nucléotide terminal 5' de la chaîne obtenue, jusqu'à obtention de la fraction d'oligonucléotide désirée.

7. Procédé d'obtention de la sonde mixte selon la revendication 4, à partir du précurseur selon l'une des revendications 1 à 3, caractérisé en ce qu'il consiste essentiellement à successivement:

a) traiter le précurseur par un mélange molaire de pyridine aldoxime (PAO) dans la tétraméthyl guanidine (TMG), additionné d'ammoniaque concentrée (à 20% au moins) dans la proportion de 1 volume de mélange PAO-TMG pour 10 volumes d'ammoniaque, à la température ambiante, pendant une nuit ;

b) traiter le mélange obtenu sous a), évaporé à sec, par l'ammoniaque concentrée, pendant environ 5 heures à environ 50°C ; et

c) traiter le mélange obtenu sous b), évaporé à sec, par l'éthylène diamine, pendant environ 72 heures à la température ambiante.

8. Utilisation de la sonde mixte selon la revendication 4 ou 5 dans le domaine des analyses et extractions biologiques, notamment de détection et d'extraction d'ARN messagers et d'ADN complémentaires, au moyen d'une hybridation.

9. Composés répondant à la formule générale

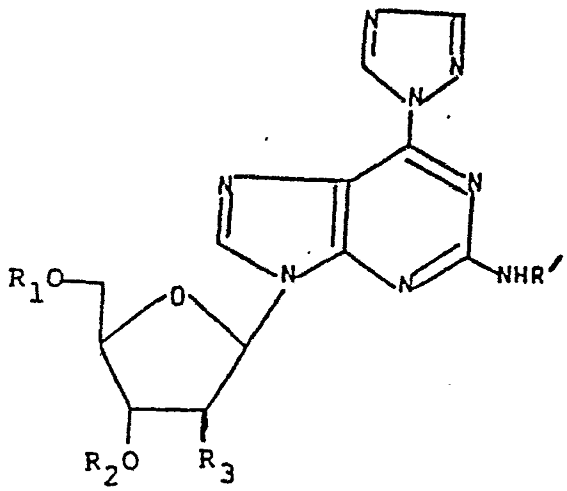

dans laquelle :

R' représente un atome d'hydrogène ou un groupe protecteur du groupe amino, notamment le groupe isobutyryle,

$R_1$ représente un atome d'hydrogène ou un groupe protecteur du groupe hydroxyle, notamment le groupe diméthoxytrityle ;

$R_2$ représente un atome d'hydrogène, un groupe protecteur du groupe hydroxyle ou un groupe phosphoryle éventuellement modifié, notamment le groupe O-(chloro-2 phényl/cyanoéthyl) phosphate ; et

$R_3$ représente un atome d'hydrogène, un groupe OH ou un groupe OH protégé, notamment le groupe benzoyloxy.

10. La N-isobutyryl-2 O-diméthoxytrityl-5' O-(chloro-2 phényl cyanoéthyl)-phosphate-3' triazolo-4 désoxy-2' guanosine.

Office européen des brevets

**RAPPORT DE RECHERCHE EUROPEENNE**

0177082 Numéro de la demande

EP 85 40 1666

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| X | JOURNAL OF ORGANIC CHEMISTRY, vol. 47, no. 19, 10 septembre 1982, pages 3623-3628, American Chemical Society, US; W.L. SUNG: "Synthesis of 4-(1,2,4-Triazol-1-yl)pyrimidin-2(1H)-one Ribonucleotide and Its Application in Synthesis of Oligoribonucleotides" * Pages 3623-3626 * --- | 1-8 | C 07 H 21/00<br>C 12 Q 1/68<br>C 07 H 19/16 |
| X,D | NUCLEIC ACIDS RESEARCH, vol. 9, no. 22, 1981, pages 6139-6151, IRL Press Ltd., Londres, GB; W.L. SUNG: "Synthesis 4-triazolopyrimidinone nucleotide and its application in synthesis of 5-methylcytosinecontaining oligodeoxyribonucleotides" * Pages 6139-6145 * --- | 1-8 | DOMAINES TECHNIQUES RECHERCHES (Int. Cl.4) |
| Y | TETRAHEDRON LETTERS, vol. 21, no. 23, 1980, pages 2265-2268, Pergamon Press Ltd., Oxford, GB; C.B. REESE et al.: "Reaction between 1-arenesulphonyl-3-nitro-1,2,4-triazoles and nucleoside base residues. Elucidation of the nature of side-reactions during oligonucleotide synthesis" * Pages 2265-2268 * --- -/- | 1-9 | C 07 H 21/00<br>C 12 Q 1/00<br>C 07 H 19/00 |

Le présent rapport de recherche a été établi pour toutes les revendications

| Lieu de la recherche | Date d'achèvement de la recherche | Examinateur |
|---|---|---|
| LA HAYE | 24-10-1985 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

# RAPPORT DE RECHERCHE EUROPEENNE

0177382

Numero de la demande

EP 85 40 1666



## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| X,Y | CHEMICAL ABSTRACTS TENTH COLLECTIVE INDEX, vol. 86-95, 1977-1981, page 4224CS, Columbus, Ohio, US; "Chemical substances Azanonaborane - Benzene, ethan" * Benzamide, N-(6-(1H-1,2,4-TRIAZOL-1-YL)-9-(2,3,5-TRI-O-BENZOYL-Beta-D-RIBOFURANOSYL)-9H-PURIN-2-YL)-[76887-36-0], 94 : 121837c * | 9 | |
| | ----- | | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 24-10-1985 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82